(19) Europäisches Patentamt
European Patent Office
Office européen des brevets

(11) EP 3 413 901 B1

(12) **EUROPEAN PATENT SPECIFICATION**

(45) Date of publication and mention of the grant of the patent:
**29.01.2020 Bulletin 2020/05**

(21) Application number: **17717246.7**

(22) Date of filing: **13.02.2017**

(51) Int Cl.:
***A61K 33/42*** *(2006.01)* ***A61K 9/00*** *(2006.01)*
***A61K 9/14*** *(2006.01)* ***A61P 19/02*** *(2006.01)*
***A61P 19/06*** *(2006.01)*

(86) International application number:
**PCT/IT2017/000027**

(87) International publication number:
**WO 2017/138031 (17.08.2017 Gazette 2017/33)**

(54) **INTRA-ARTICULAR ADMINISTRATION OF POLYMETAPHOPSPHATES FOR THE TREATMENT OF CRYSTAL ARTHROPATIES**

INTRAARTIKULÄRE VERABREICHUNG VON POLYMETAPHOPSPHATEN ZUR BEHANDLUNG VON KRISTALLINEN ARTHROPATHIEN

ADMINISTRATION INTRA-ARTICULAIRE DE POLYMÉTAPHOSPHATES POUR LE TRAITEMENT D'ARTHROPATHIES CRISTALLINES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.02.2016 IT UB20160717**

(43) Date of publication of application:
**19.12.2018 Bulletin 2018/51**

(73) Proprietors:
- **Over S.r.l.**
  **53100 Siena (SI) (IT)**
- **Laboratorio Farmacologico Milanese S.r.l.**
  **21042 Caronno Pertusella (VA) (IT)**

(72) Inventors:
- **FIGURA, Natale**
  **53040 Rapolano Terme (SI) (IT)**
- **CAVALLO, Giovanni**
  **122 Roma (IT)**
- **PANZERI, Ivo**
  **21042 Caronno Pertusella (VA) (IT)**

(74) Representative: **Banchetti, Marina et al**
**Barzanò & Zanardo Roma S.p.A.**
**Via Piemonte 26**
**00187 Roma (IT)**

(56) References cited:
**WO-A2-2005/060978**

- **CINI R ET AL: "Dissolution of calcium pyrophosphate crystals by polyphosphates: An in vitro and ex vivo study", ANNALS OF THE RHEUMATIC DISEASES, BRITISH MEDICAL ASSOCIATION, GB, vol. 60, no. 10, 1 October 2001 (2001-10-01), pages 962-967, XP002328845, ISSN: 0003-4967, DOI: 10.1136/ARD.60.10.962 cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 413 901 B1

Printed by Jouve, 75001 PARIS (FR)

## Description

### Field of the invention

The present invention relates to some polymetaphosphates for intra-articular administration for use in the treatment of crystal arthropaties. More particularly, the invention concerns preparations based on a sodium polyphosphate of a defined structure for the continuous or step-wise articular lavage for use in the treatment of arthropathies due to microcrystal deposition, originated by calcium deposits in the articular tissues.

### Background of the invention

As it is known, microcrystalline arthropathies are a group of inflammatory-degenerative pathologies, characterized by the deposition of mineral substances in articular and periarticular structures in crystalline form. In particular. chondrocalcinosis, also known as pseudogout, is a disease characterized by microcrystalline deposits of calcium pyrophosphate dihydrate (CPPD), having the formula $Ca_2[O(PO_3)_2](2H_2O)$, and having the following structure:

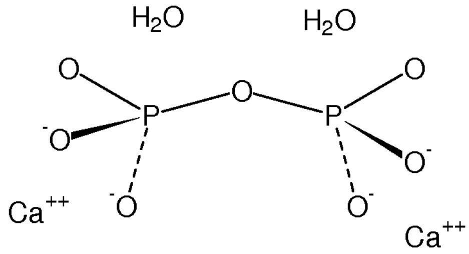

In the course of chondrocalcinosis, synovitic episodes secondary to the release of CPPD crystals from tissue deposits in the synovial frequently occur. The identification of crystals in the synovial liquid of patients with gout-like arthritis was described in 1962 by McCarthy [McCarthy DJ Jr et al., The significance of calcium phosphate crystal in the synovial fluid of arthritis patients: the pseudogout syndrome. Clinical aspects. Ann Intern Med 1962, 56:711-737).

Another common microcrystalline arthropathy is caused by the deposition at the articular and periarticular level of hydroxyapatite crystals, $Ca_5(PO_4)_3OH$, (HAP), the structure of which may be schematically represented as follows:

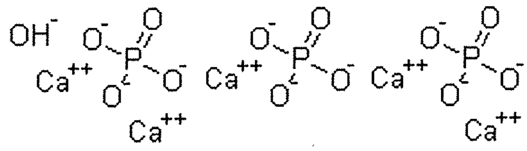

Usually, the arthropathy from hydroxyapatite deposition manifests itself in association with other arthropathies of a mainly degenerative nature, such as osteoarthrosis, calcific periarthritis, tendinitis and calcific bursitis. Although calcific deposits are often not associated with specific clinical signs, they can assume particular relevance in conditions such as calcific periarthritis of the shoulder, where it is believed that such calcifications are partly responsible for the inflammatory degenerative manifestations of the periarticular structure (Dieppe PA, et al., Apatite deposition disease: a new arthropathy. Lancet 1976, 1:266-268)

The mechanism that leads to the precipitation and deposition of CPPD or HAP crystals is not yet known, nor does it appear clear whether the degenerative alterations of the cartilage are primitive or secondary to the deposition of the crystals. The likeliest hypothesis is that this deposition is due to a local metabolic alteration. In case of chondrocalcinosis, the pyrophosphate produced by the chondrocytes would be diffused in the fundamental substance due to an increased synthesis or to an inability of the tissue to hydrolyze the compound with pyrophosphatase enzymes, including alkaline phosphatase. Small deposits of pyrophosphate are often observed in the cartilage of elderly subjects, especially as a result of an increased synthesis and concentration of pyrophosphates, due to nucleoside triphosphate pyrophosphohydrolase (NTPPPH) enzymes (Ryan ML et al., Calcium Pyrophosphate Crystal Deposition Disease; Pseudogout; Articular Chondrocalcinosis. In: Arthritis and Allied Conditions: A Textbook of Rheumatology (D.J. McCarthy & W.J. Koopman eds.), vol. 2 (12th ed.), Philadelphia, Pa, Lippincott Williams & Wilkins, 1993, pp. 1835-1855).

In turn, pyrophosphates are an important source of inorganic phosphates, which have a fundamental role in bone mineralization. There is an equilibrium between pyrophosphates and phosphates: when the former prevail, they precipitate in crystalline form; when phosphates prevail, there is a greater solubilization and reduction of pyrophosphate

crystals (Anderson HC, Mechanisms of pathologic calcification. Rheum Dis Clin N Am 1988, 14:303-319).

CPPD crystals have elongated rhomboidal shape, although at times they are evidenced in the form of long or short rods and small squares, whereas HAP crystals are smaller and have needle or rod shape. Currently, it is believed that acute pseudogout attacks are due to the release into the articular cavity (synovial liquid) of CPPD crystals, which are coated (opsonized) with proteins (especially IgG) and then recognized and phagocytosed by polymorphonuclear neutrophils (PMN). During phagocytosis and the subsequent cell destruction lysosomal enzymes, reactive oxygen species (ROS), and leukotrienes are released which act as chemical mediators of the inflammation, with consequent acute arthritis or pseudogout (Burt HM, Jackson JK. Enhancement of crystal induced neutrophil responses by opsonisation of calcium pyrophosphate dihydrate crystals. Ann Rheum Dis 1993, 52: 599-607).

It is supposed that shape, size and amount of the crystals play quite specific roles in PMN activation. On this subject, there are numerous studies which, while confirming the phlogogenic activity of CPPD crystals, are in poor agreement above all on the size of the crystalline material able to stimulate phagocytes more intensely (Shwan A et al., Comparison of sodium urate and calcium pyrophosphate crystal size and other factors. Arthritis Rheum 1995, 18 (suppl):783-793).

At the moment, only symptomatic therapies to reduce acute pseudogout attacks are available, and they are often unable to result in a durable effect. The most widely used treatment for the acute form consists of performing an arthrocentesis on the inflamed articulation, possibly associated to articular washing with physiological solution and/or local infiltration of corticosteroids (Fitzgerald RH Jr. Intrasynovial injection of steroids uses and abuses, 1976, Mayo Clin Proc 51:655-659; Werlen O et al., Corticosteroid therapy for the treatment of acute attacks of crystal-induced arthritis: an effective alternative to nonsteroidal antiinflammatory drugs. Rev Rhum Engl Ed 1996, 63:248-254).

Alternatively or in association with the aforesaid therapy, non steroidal anti-inflammatory drugs and/or colchicines are used, although the problem of the persistence of CPPD or HAP crystals at the tissue level still remains (Abramson SB. Treatment of gout and crystal arthropathies and use and mechanisms of action of nonsteroidal anti-inflammatory drugs. Curr Opin Rheumatol 1992, 4:295-300).

In regards to prophylaxis, currently, the only prophylaxis for pseudogout attacks is the use of oral colchicine (Gonzales T, Gantes M. Prevention of acute attacks of pseudogout with oral colchicine. J Rheumatol 1987, 14 632-633; Lange U et al., Current aspects of colchicine therapy - classical indications and new therapeutic uses. Eur J Med Res 2001, 6:150-160).

In the case of CPPD crystals, approaches have been attempted using the enzymatic route, i.e. the enzymes that are able to degrade pyrophosphates, such as yeast phosphatase and alkaline phosphatase. However, these attempts have not found a valid therapeutic application, presumably due to the difficulty of preparing adequate formulations of protein origin because of antigen problems and of the high costs of production (Xu Y et al., Effects of pyrophosphatase on dissolution of calcium pyrophosphate dihydrate crystals. J Rheumatol, 1991. 18:66-71; Shinozaki T et al., Calcium pyrophosphate dihydrate (CPPD) crystal dissolution by alkaline phosphatase: interaction of alkaline phosphatase on CPPD crystals. J Rheumatol 1995, 22:117-123).

As previously mentioned, the pathogenic action of HAP crystals in the development of articular inflammatory events is quite unclear, although crystalline aggregates of HAP are frequently present in articular effusions, both of inflammatory and degenerative nature, so that their presence is considered an epiphenomenon. On the contrary, the action of hydroxyapatite crystal deposits in the development of periarticular inflammatory degenerative pathologies, such as calcific periarthritis, clinically expressed in acute and/or chronic painful shoulder conditions, is well known. Currently, there are treatments aimed at the destruction and/or removal of such microcrystalline deposits, such as articular washings with physiological solution and Extracorporeal Shock Wave Therapy (ESWT) (Cosentino R. et al., Extracorporeal shock wave therapy for chronic calcific tendinitis of the shoulder: single blind study. Ann Rheum Dis 2003, 62:248-50; Ebenbichler GR, et al., Ultrasound therapy for calcific tendinitis of the shoulder. N Engl J Med 1999, 341: 1237).

The activity of polymetaphosphates to antagonize the crystallization of calcium-based salts (e.g. calcium carbonate and calcium sulfate) and other metals (e.g. iron, magnesium) is known since long time. This class of compounds, therefore, finds widespread use in the production of softeners of hard and industrial waters, detergents in textile industries and/or dispersing agents in fabric coloring operations. In cosmetics, polymetaphosphates are particularly effective in the treatment of calcium deposits as they are important ingredients in anti-plaque toothpastes.

The *In vitro* solubilizing ability of some polymetaphosphates on CPPD aggregates had already been described at a preliminary experimental level in 2001 (Cini R. et al., Dissolution of calcium pyrophosphate crystals by polyphosphates: an in vitro and ex vivo study. Ann Rheum Dis 2001, 60:962-967), and has been fully described in the international patent application publication No. WO 2005/060978 (Università degli Studi di Siena).

This document describes preparations based on linear or cyclic polymetaphosphate salts, preferably sodium salts of general formula $(Na\text{-}PO_3)_n$, as products per se, as well as their use for intra-articular treatment of microcrystalline arthropathy, preferably in combination with antioxidants and/or anti-free radical substances (scavengers). Preferred polymetaphosphates are those selected from the following group: polymeric sodium metaphosphate (also known as sodium metaphosphate, SMP, formula a); sodium tripolymetaphosphate or tripolyphosphate (STPP or, according IUPAC

nomenclature, pentasodium triphosphate: PSTP, formula b); cyclic sodium trimetaphosphate or trisodium metaphosphate (TSMP, formula c); cyclic hexametaphosphate (SEMP or SHMP, formula d).

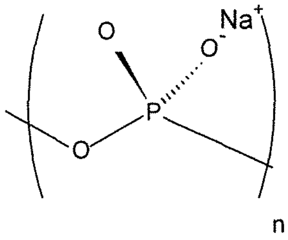

(a) SMP, $(NaPO_3)_n$

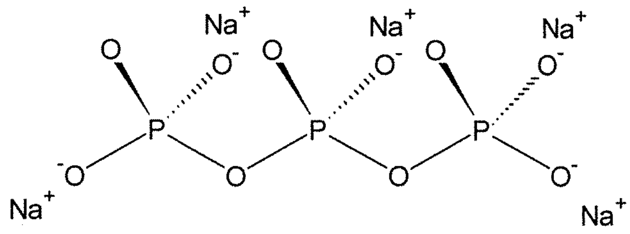

(b) STPP (or PSTP), $Na_5P_3O_{10}$

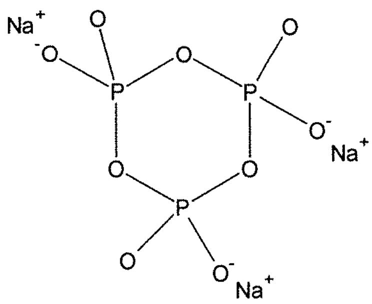

(c) TSMP, $Na_3P_3O_9$

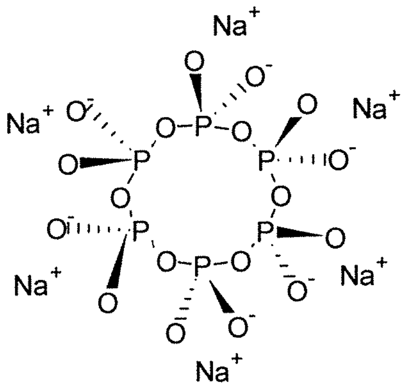

(d) SEMP (or SHMP), $Na_6P_6O_{18}$

According to the cited document, the described linear or cyclic polymetaphosphates have shown a high dissolving capacity with respect to microcrystalline calcific deposits based on calcium pyrophosphate or hydroxyapatite, and therefore they are proposed for use the treatment of microcrystalline arthropathies. The proposed treatment consists in the intra-articular injection of aqueous solutions based on one of the mentioned compounds, with the purpose of solubilizing the deposits of CPPD or HAP.

Although the international publication WO 2005/060978 also refers generically to operations of "continuous lavage" with the same substances, any indication and experimentation in the document only relates to the intra-articular injection of solutions based on of the above substances with amounts of solution comprised between 5 and 10 ml, and designed to achieve a "static" solubilization of calcific deposits by exploiting the chelating properties of calcium that the claimed substances were known to possess. In the hypothesis, albeit theoretical, of using "continuous lavage", a volume of solution (with unspecified concentration) ranging from 5 to 50 ml is proposed.

**Summary of the invention**

After having experimentally verified the results, in terms of dissolution of the accumulations of calcium pyrophosphate dihydrate or hydroxyapatite crystals - and, thus, in terms of remission of the pain symptoms due to the presence of such accumulations in joint tissue - which can be obtained with the intra-articular injection of products based on polymetaphosphates of the prior art, the object set was to improve those results, by implementing administration procedures more effective than those previously described.

The proper articular lavage carried out continuously with remarkable volumes of physiological solution is a known technique, used in arthritic pathologies. In such case the objective is not to dissolve calcified deposits present in the synovial fluid and in the articular district, but to reduce the painful symptoms linked to the evolution of an arthritic degeneration, by removing from the articulation slag and debris that normally disturb the joint mechanics.

According to the present invention, it has been found that by applying to arthropathies due to microcrystals deposition a dosage regimen of a polymetaphosphate-based drug more similar to that of the articular lavage that is performed for arthritic diseases, a mechanical washing action is associated to the chelating and solubilizing of polymetaphosphate. This additional mechanical action synergistically increases the results obtainable with the intraarticular injection of a small amount of solution for the "static" dissolution of calcific deposits, as expected from the prior art.

Therefore, in order to treat chondrocalcinosis, and in general microcrystalline arthropathies, there is proposed to use the same agents with deposits-dissolving activity already known, but used in a modality which exploits the synergism observed between a real washing action and a dissolving action. Such modality involves the application of polymetaphosphate dissolved in suitable ratios with the diluting solution, and the treatment with suitable volumes of the resulting preparation, according to a real continuous articular lavage, or better, a step-wise lavage, in which subsequent portions of product are introduced into the joint space, allowed to work and then expelled in sequence, until exhaustion of the washing solution..

**Detailed description of the invention**

The present invention, thus, specifically provides a preparation for use in the treatment of arthropathies due to microcrystal deposition, comprising a diluent aqueous sterile solution and a linear polymetaphosphate of the formula $M_{n+2}P_nO_{3n+1}$, or a cyclic polymetaphosphate of the formula $(MPO_3)_n$, wherein M represents an alkaline metal, as sterile lyophilized powder, wherein the volume of diluent solution is from 100 ml to 1500 ml and the weight/volume ratio of the lyophilized polymetaphosphate powder to the diluent solution is from 1:1000 to 10:1000, and wherein said treatment is carried out, upon reconstitution of the polymetaphosphate powder in said diluent solution,

a) by step-wise lavage, i.e. by using, for an articular lavage, a plurality of defined portions of said reconstituted solution, in sequence; or
b) by a continuous lavage with said reconstituted solution,

wherein said polymetaphosphate is selected from polymeric methaphosphate, tripolymetaphosphate and cyclic hexametaphosphate.

The polymetaphosphate according to the invention is an alkaline metal metaphosphate salt, and preferably the sodium salt; more specifically, it is a linear salt of the formula $Na_{n+2}P_nO_{3n+1}$, or a cyclic salt of the formula $(NaPO_3)_n$. Said metaphosphate salt is preferably selected from the group consisting in: sodium polymeric metaphosphate (SMP, of the formula $Na_{n+2}P_nO_{3n+1}$, with n generally comprised between 4 and 100, preferably between 30 and 90), sodium tripolymetaphosphate (STPP o PSTP, of the formula $Na_5P_3O_{10}$) and cyclic sodium hexametaphosphate (SHMP, of the formula $Na_6P_6O_{18}$), also known as Graham salt.

As already described in the cited prior art, the proposed lavage preparation may further comprise effective amounts of antioxidants and/or anti-free radical agents, such as, in particular, mannitol, known to be a powerful scavenger of hydroxyl radicals (reactive oxygen species, or ROS), vitamin E, vitamin C, carotenoids, tocopherol, taurine, glucosamine sulfate and glucosamine hydrochloride, all known antioxidants and/or ROS scavengers.

According to some embodiments of the invention, the weight/volume ratio of lyophilized polymetaphosphate powder to diluent solution ranges from 3:1000 to 7:1000, and according to a preferred solution it is equal to 5:1000.

According to other embodiments, the volume of the diluent solution ranges from 300 ml to 1000 ml, and according to a preferred solution it is equal to 500 ml.

In particular, according to a preferred embodiment, said poly-metaphosphate is sodium hexametaphosphate (SHMP), and the weight/volume ratio of lyophilized polymetaphosphate powder to diluent solution is 5:1000, while the volume of diluent solution is 500 ml.

A preferred diluent solution also comprises mannitol and phosphate buffer.

The following formulation is therefore an example of the composition of a lavage solution for use according to the invention:

| Formula A<br>Components | % Concentration (w/v) | M |
|---|---|---|
| Sodium hexametaphosphate | 0.5 | 8.17 mM |
| Monobasic potassium phosphate | 0.12 | |
| Dibasic sodium phosphate | 0,69 | |
| Potassium chloride | 0.12 | |
| Mannitol | 1.55 | |
| pH | 7.21 | |

Some experimental results that have identified the synergistic features of the application of SHMP as solubilizing

agent for calcific deposits and as an agent for mechanical lavage in the removal of residual material from the joint tissues are described below.

**Evaluation and quantification of the solubilization process of calcium pyrophosphate and hydroxyapatite by sodium hexametaphosphate (SHMP)**

The solubilization process of the two salts of **calcium hydroxyapatite** and **calcium pyrophosphate,** here at issue, has been studied in two different modality which are the subject of this invention:

a) **"Continuous lavage":** Through a peristaltic pump, the sodium hexametaphosphate solution was passed through a cylindrical chamber (hold at 37°C), containing the salt to be dissolved (calcium pyrophosphate or hydroxyapatite), with a continuous flow. The outlet chamber is equipped with a filter which allows the exit of the solution but not the salt. After the passage in the chamber, the solution was collected in flasks having known volume and analyzed to determine the quantity of dissolved calcium.
b) **"Step-wise lavage":** Through a peristaltic pump, the hexametaphosphate solution was passed in the chamber containing the salt to be dissolved (calcium pyrophosphate or hydroxyapatite); the solution was held in the chamber for 10 minutes and then replaced with the "fresh" hexametaphosphate solution. The various portions of solution were collected in flasks having known volume and analyzed to determine the quantity of dissolved calcium.
c) The same operations were carried out by passing pure water in the chamber for analysis in order to check whether there may be a "washing effect" of the salts by simple mechanical removal.

Preparation of working solutions

A solution of sodium hexametaphosphate 0,5% by weight in sterile solution, called "Safedia 0.5%", was used, reconstituted by taking out first 10 cc of solution of the product from the bag with a sterile syringe and introducing it into the vial of freeze-dried sodium hexametaphosphate, shaking the preparation in the vial, and then withdrawing the solution obtained from the bottle with a new syringe, injecting the solution into the bag and finally shaking the latter.

Experimental conditions:

**Continuous lavage:**

- 500 ml of solution were passed (pure water for analysis or "Safedia 0.5 %" solution), in the cylindrical chamber equipped with output filter containing 50.0 mg of salt to be dissolved (calcium pyrophosphate o hydroxyapatite) ;
- the chamber was thermalized at 37° C, through a heat bath;
- the passage of the solution flow was adjusted to 8 ml/min;
- 5 portions 100 ml each of solution "Safedia 0.5%" were collected out of the chamber,.

**Step-wise lavage:**

- 100 ml of solution (pure water for analysis or solution "Safedia 0.5%") of 50.0 mg salt to be dissolved (calcium pyrophosphate o hydroxyapatite) were placed in the cylindrical chamber equipped with output filter;
- the solution remained in contact with the salt for 10 minutes;
- then the solution was replaced with 100 ml of fresh solution "Safedia 0.5%";
- these steps were repeated 5 times;
- the room was thermalized at 37 ° C, through a heat bath;
- 5 portions 100 ml each of solution "Safedia 0.5%" were collected out of the chamber.

Analysis of the outgoing solutions

All the portions of outgoing solutions were analyzed for atomic absorbance, in order to verify the presence of calcium.
The method used was APAT IRSA CNR 3130

Calculation and results

*Dissolving solution: water - Salt to be dissolved: calcium pyrophosphate*

*Modality: Continuous lavage*

Considering that in calcium pyrophosphate (CPPD) 31.48% of $Ca^{++}$ is present and that in the chamber 500 mg of CPPD have been placed, it follows that in the chamber were present:

$$500*0.3148= 157.4 \text{ mg } Ca^{++}$$

The outgoing calcium found in the 5 solution portions outgoing from the chamber is reported in the following table:

| Portions of water | $Ca^{++}$ mg |
|---|---|
| 1 | 0.09 |
| 2 | 0.08 |
| 3 | 0.07 |
| 4 | 0.07 |
| 5 | 0.06 |

Therefore, in total, the water solution in continuous lavage modality removed:

$$0.09+0.08+0.07+0.07+0.06= 0.39 \text{ mg } Ca^{++}$$

corresponding to:

0.39/157.4 = **0.23% of calcium brought in solution**.

*Dissolving solution: water- Salt to be dissolved: hydroxyapatite*

*Modality: Continuous lavage*

Considering that in calcium hydroxyapatite (HAP) 39.82% of $Ca^{++}$ is present and that 500 mg of HAP was placed in the chamber, it follows that the chamber contained:

$$500*0.3982= 199.1 \text{ mg } Ca^{++}$$

The outgoing calcium found in the 5 solution portions outgoing from the chamber is reported in the following table:

| Portions of water | $Ca^{++}$ mg |
|---|---|
| 1 | 0.04 |
| 2 | 0.04 |
| 3 | 0.03 |
| 4 | 0.03 |
| 5 | 0.02 |

Therefore, in total the water solution in continuous lavage modality removed:

$$0.04+0.04+0.03+0.03+0.02= 0.16 \text{ mg } Ca^{++}$$

corresponding to:

0.16/199.1 = **0.08% of calcium brought in solution**

*Dissolving solution: Safedia 0.5% - Salt to be dissolved: calcium pyrophosphate*

*Modality: Continuous lavage*

The outgoing calcium found in the 5 solution portions outgoing from the chamber is reported in the following table:

| Portions of Safedia 0.5% | $Ca^{++}$ mg |
|---|---|
| 1 | 15,6 |
| 2 | 14.9 |
| 3 | 14.8 |
| 4 | 14.2 |
| 5 | 13.7 |

Therefore, in total the water solution in continuous lavage modality removed:

$$15,6+14.9+14.8+14.2+13.7= 73.2 \text{ mg } Ca^{++}$$

corresponding to:

73.2/157.4 = **46.5% of calcium brought in solution**

*Dissolving solution: Safedia 0.5% - Salt to be dissolved: hydroxyapatite*

*Modality: Continuous lavage*

The outgoing calcium found in the 5 solution portions outgoing from the chamber is reported in the following table:

| Portions of Safedia 0.5% | $Ca^{++}$ mg |
|---|---|
| 1 | 6.3 |
| 2 | 6.0 |
| 3 | 5.9 |
| 4 | 5.8 |
| 5 | 5,6 |

Therefore, in total the water solution in continuous lavage modality removed:

$$6.3+6.0+5.9+5.8+5,6= 29,6 \text{ mg } Ca^{++}$$

corresponding to:

$$29,6/199.1 = \textbf{14.8\% of calcium brought in solution}$$

*Dissolving solution: water - Salt to be dissolved: calcium pyrophosphate*

*Modality: Step-wise lavage*

The outgoing calcium found in the 5 solution portions outgoing from the chamber is reported in the following table:

| Portions of water | $Ca^{++}$ mg |
|---|---|
| 1 | 0.11 |
| 2 | 0.10 |
| 3 | 0.08 |
| 4 | 0.08 |
| 5 | 0.06 |

Therefore, in total the water solution in continuous lavage modality removed:

$$0.11+0.10+0.08+0.08+0.06= 0.43 \text{ mg } Ca^{++}$$

corresponding to:

$$0.43/157.4 = \textbf{0.27\% of calcium brought in solution}$$

*Dissolving solution: water - Salt to be dissolved: hydroxyapatite*

*Modality: Step-wise lavage*

The outgoing calcium found in the 5 solution portions outgoing from the chamber is reported in the following table:

| Portions of water | $Ca^{++}$ mg |
|---|---|
| 1 | 0.05 |
| 2 | 0.05 |
| 3 | 0.05 |
| 4 | 0.04 |
| 5 | 0.04 |

Therefore, in total, the water solution in continuous lavage modality removed:

$$0.05+0.05+0.05+0.04+0.03= 0.22 \text{ mg } Ca^{++}$$

corresponding to:

$$0.22/199.1 = \textbf{0.11\% of calcium brought in solution}$$

*Dissolving solution: Safedia 0.5% - Salt to be dissolved: calcium pyrophosphate*

*Modality: Step-wise lavage*

The outgoing calcium found in the 5 solution portions outgoing from the chamber is reported in the following table:

| Portions of Safedia 0.5% | $Ca^{++}$ mg |
|---|---|
| 1 | 16.8 |
| 2 | 16.8 |
| 3 | 16.5 |
| 4 | 16.3 |
| 5 | 16.3 |

Therefore, in total, the water solution in continuous lavage modality removed:

$$16.8+16.8+16.5+16.3+16.3= 82.7 \text{ mg } Ca^{++}$$

corresponding to:

82.7/157.4 = **52.5% of calcium brought in solution**

*Dissolving solution: Safedia 0.5% - Salt to be dissolved: hydroxyapatite Modality: Step-wise lavage*

The outgoing calcium found in the 5 solution portions outgoing from the chamber is reported in the following table:

| Portions of Safedia 0.5% | $Ca^{++}$ mg |
|---|---|
| 1 | 8.2 |
| 2 | 8.1 |
| 3 | 7,6 |
| 4 | 7.3 |
| 5 | 6.9 |

Therefore, in total the water solution in continuous lavage modality removed:

$$8.2+8.1+7,6+7.3+6.9= 38.1 \text{ mg } Ca^{++}$$

corresponding to:

38.1/199.1 = **19.1% of calcium brought in solution**

The results are summarized in the following table:

| Dissolving solution | Salt to be dissolved | Modality | % Calcium brought in solution |
|---|---|---|---|
| Water | Calcium pyrophosphate | Continuous lavage | 0.23 |
| Water | Hydroxyapatite | Continuous lavage | 0.08 |
| Safedia 0.5% | Calcium pyrophosphate | Continuous lavage | 46.5 |

(continued)

| Dissolving solution | Salt to be dissolved | Modality | % Calcium brought in solution |
|---|---|---|---|
| Safedia 0.5% | Hydroxyapatite | Continuous lavage | 14.8 |
| Water | Calcium pyrophosphate | Step-wise lavage | 0.27 |
| Water | Hydroxyapatite | Step-wise lavage | 0.11 |
| Safedia 0.5% | Calcium pyrophosphate | Step-wise lavage | 52.5 |
| Safedia 0.5% | Hyd roxyapatite | Step-wise lavage | 19.1 |

Comparison with previous data

For a comparison with the prior art described by the earlier patent application WO2005/060978 a series of measures to check whether a hexametaphosphate solution could dissolve salts of calcium pyrophosphate and hydroxyapatite in "static" fashion were carried out.

50 mg of the two of calcium to be dissolved with 50 ml of Safedia 0.5% solution were placed in a beaker, the mixture was put under stirring for 60 minutes and held at 37°C by thermostatic bath.

The salts to be dissolved and the solvent used were the same as used in this disclosure.

The results obtained by operating according to the mentioned patent application are reported in the following table:

| Dissolving solution | Salt to be dissolved | Modality | % calcium brought in solution |
|---|---|---|---|
| Safedia 0.5% | Calcium pyrophosphate | Static solubilization | 30.6 |
| Safedia 0.5% | Hydroxyapatite | Static solubilization | 10.4 |

As can be seen from the above two tables the solubilization of calcium pyrophosphate in static lavage modality is 30,6%, compared to 46.5% in continuous lavage modality, to reach 52.5% in modality step-wise lavage.

For hydroxyapatite the solubilization in static lavage modality is 10.4% compared to 14.8% in continuous lavage modality, to reach 19.1% in step-wise lavage modality.

The foregoing shows that in continuous lavage and step-wise lavage modality the solubility of the two calcium salts is greater than in the static lavage modality, because a mechanical removal effect of the two salts is added to the effect of chemical solubilization by Safedia 0.5% solution.

**Evaluation and quantification of the solubilization process of calcium pyrophosphate and hydroxyapatite by a solution of sodium metaphosphate (SMP) and a solution of sodium tripolymetaphosphate (STPP)**

The same experiment performed to the sodium hexametaphosphate (SHMP) above was repeated to verify the solubilization process of calcium pyrophosphate and of hydroxyapatite by solutions of SMP and STPP.

Preparation of working solutions

- 0,5% solution of SMP: 5.000 g of sodium metaphosphate (SMP) were weighed, decanted into a 1000 ml volumetric flask containing about 500 ml of water. The salt has been completely solubilized by shaking and then the flask was brought to volume.
- 0,5% solution of STPP: the same procedure was repeated starting from 5.000 g of sodium tripolyphosphate.

Experimental conditions:

**Continuous lavage:**

The same conditions of the previous experiments were reproduced with 500 ml of pure water for analysis, or 0.5% solution of SMP, or 0.5% solution of STPP.

**Step-wise lavage:**

The same conditions of the previous experiments were reproduced with 500 ml of pure water for analysis, or 0.5% solution of SMP, or 0.5% solution of STPP.

Analysis of the outgoing solutions

All the portions of outgoing solutions have been analyzed for atomic absorbance, in order to verify calcium presence.

The methodology used was: APAT IRSA CNR 3130

Calculation and results

*Dissolving solution: water - Salt to be dissolved: calcium pyrophosphate*

*Modality: Continuous lavage*

Considering that in calcium pyrophosphate (CPPD) 31.48% of $Ca^{++}$ is present and that in the chamber 500 mg of CPPD have been placed, it follows that in the chamber were present:

$$500*0.3148= 157.4 \text{ mg } Ca^{++}$$

The outgoing calcium found in the 5 solution portions outgoing from the chamber is reported in the following table:

| Portions of water | $Ca^{++}$ mg |
|---|---|
| 1 | 0.10 |
| 2 | 0.10 |
| 3 | 0.08 |
| 4 | 0.07 |
| 5 | 0.07 |

Therefore, in total the water solution in continuous lavage modality removed:

$$0.10+0.10+0.08+0.07+0.07= 0.42 \ Ca^{++} \text{ mg}$$

corresponding to:

0.42/157.4 = **0.27% of calcium brought in solution**.

*Dissolving solution: water - Salt to be dissolved: hydroxyapatite*

*Modality: Continuous lavage*

Considering that in calcium hydroxyapatite (HAP) 39.82% of $Ca^{++}$ is present and that in the chamber 500 mg of HAP have been placed, it follows that in the chamber were present:

$$500*0.3982= 199.1 \text{ mg } Ca^{++}$$

The outgoing calcium found in the 5 solution portions outgoing from the chamber is reported in the following table:

| Portions of water | $Ca^{++}$ mg |
|---|---|
| 1 | 0.04 |
| 2 | 0.04 |
| 3 | 0.04 |
| 4 | 0.04 |
| 5 | 0.03 |

Therefore, in total the water solution in continuous lavage modality removed:

$$0.04+0.04+0.04+0.04+0.03 = 0.19 \text{ mg } Ca^{++}$$

corresponding to:

0.19/199.1 = **0.10% of calcium brought in solution**

*Dissolving solution: Sodium metaphosphate 0.5% - Salt to be dissolved calcium pyrophosphate*

*Modality: Continuous lavage*

The outgoing calcium found in the 5 solution portions outgoing from the chamber is reported in the following table:

| Portions of SMP 0.5% | $Ca^{++}$ mg |
|---|---|
| 1 | 11.8 |
| 2 | 11.5 |
| 3 | 10.7 |
| 4 | 10.3 |
| 5 | 9.7 |

Therefore, in total the water solution in continuous lavage modality removed:

$$11.8+11.5+10.7+10.3+9.7 = 54.2 \text{ mg } Ca^{++}$$

corresponding to:

54.2/157.4 = **34.4% of calcium brought in solution**

*Dissolving solution: Sodium metaphosphate 0.5% - Salt to be dissolved: hydroxyapatite*

*Modality: Continuous lavage*

The outgoing calcium found in the 5 solution portions outgoing from the chamber is reported in the following table:

| Portions of SMP 0.5% | $Ca^{++}$ mg |
|---|---|
| 1 | 4.2 |
| 2 | 4.1 |

(continued)

| Portions of SMP 0.5% | $Ca^{++}$ mg |
|---|---|
| 3 | 4.1 |
| 4 | 3.8 |
| 5 | 3.5 |

Therefore, in total the water solution in continuous lavage modality removed:

$$4.2+4.1+4.1+3.8+3.5= 19.7 \text{ mg } Ca^{++}$$

corresponding to:

19.7/199.1 = **9.9% of calcium brought in solution**

*Dissolving solution: Sodium tripolyphosphate 0.5% - Salt to be dissolved: calcium pyrophosphate*

*Modality: Continuous lavage*

The outgoing calcium found in the 5 solution portions outgoing from the chamber is reported in the following table:

| Portions of STPP 0.5% | $Ca^{++}$ mg |
|---|---|
| 1 | 9.4 |
| 2 | 9.1 |
| 3 | 8.7 |
| 4 | 8.2 |
| 5 | 8.1 |

Therefore, in total the water solution in continuous lavage modality removed:

$$9.4+9.1+8.7+8.2+8.1= 43.5 \text{ mg } Ca^{++}$$

corresponding to:

43.5/157.4 = **27.6% of calcium brought in solution**

*Dissolving solution: Sodium tripolyphosphate 0.5% - Salt to be dissolved hydroxyapatite*

*Modality: Continuous lavage*

The outgoing calcium found in the 5 solution portions outgoing from the chamber is reported in the following table:

| Portions of STPP 0.5% | $Ca^{++}$ mg |
|---|---|
| 1 | 3.3 |
| 2 | 3.2 |
| 3 | 2.6 |
| 4 | 2.4 |

(continued)

| Portions of STPP 0.5% | $Ca^{++}$ mg |
|---|---|
| 5 | 2.4 |

Therefore, in total the water solution in continuous lavage modality removed:

$$3.3+3.2+2.6+2.4+2.4= 14.2 \text{ mg } Ca^{++}$$

corresponding to:

$$14.2/199.1 = \mathbf{7.1\% \text{ of calcium brought in solution}}$$

*Dissolving solution: water - Salt to be dissolved: calcium pyrophosphate*

*Modality: Step-wise lavage*

The outgoing calcium found in the 5 solution portions outgoing from the chamber is reported in the following table:

(Table follows)

| Portions of water | $Ca^{++}$ mg |
|---|---|
| 1 | 0.13 |
| 2 | 0.13 |
| 3 | 0.12 |
| 4 | 0.11 |
| 5 | 0.11 |

Therefore, in total the water solution in continuous lavage modality removed:

$$0.13+0.13+0.12+0.11+0.11= 0.48 \text{ mg } Ca^{++}$$

corresponding to:

$$0.48/157.4 = \mathbf{0.30\% \text{ of calcium brought in solution}}$$

*Dissolving solution water - Salt to be dissolved hydroxyapatite*

*Modality: Step-wise lavage*

The outgoing calcium found in the 5 solution portions outgoing from the chamber is reported in the following table:

| Portions of water | $Ca^{++}$ mg |
|---|---|
| 1 | 0.07 |
| 2 | 0.07 |
| 3 | 0.06 |
| 4 | 0.06 |
| 5 | 0.04 |

Therefore, in total the water solution in continuous lavage modality removed:

$$0.07+0.07+0.06+0.06+0.04= 0.30 \text{ mg } Ca^{++}$$

corresponding to:

$$0.30/199.1 = \mathbf{0.15\% \text{ of calcium brought in solution}}$$

*Dissolving solution: Sodium metaphosphate 0.5% - Salt to be dissolved: calcium pyrophosphate*

*Modality: Step-wise lavage*

The outgoing calcium found in the 5 solution portions outgoing from the chamber is reported in the following table:

| Portions of SMP 0.5% | $Ca^{++}$ mg |
|---|---|
| 1 | 12.0 |
| 2 | 11.9 |
| 3 | 11.7 |
| 4 | 11.6 |
| 5 | 11.4 |

Therefore, in total the water solution in continuous lavage modality removed:

$$12.0+11.9+11.7+11.6+11.4 = 58.6 \text{ mg } Ca^{++}$$

corresponding to:

$$58.6/157.4 = \mathbf{37.2\% \text{ of calcium brought in solution}}$$

*Dissolving solution: Sodium metaphosphate 0.5% - Salt to be dissolved: hydroxyapatite Modality: Step-wise lavage*

The outgoing calcium found in the 5 solution portions outgoing from the chamber is reported in the following table:

| Portions of SMP 0.5% | $Ca^{++}$ mg |
|---|---|
| 1 | 4.8 |
| 2 | 4.8 |
| 3 | 4.5 |
| 4 | 4.3 |
| 5 | 4.2 |

Therefore, in total the water solution in continuous lavage modality removed:

$$4.8+4.8+4.5+4.3+4.2 = 22.6 \text{ mg } Ca^{++}$$

corresponding to:

$$22.6/199.1 = \mathbf{11.4\%\ of\ calcium\ brought\ in\ solution}$$

*Dissolving solution: Sodium tripolyphosphate 0.5% - Salt to be dissolved: calcium pyrophosphate*

*Modality: Step-wise lavage*

The outgoing calcium found in the 5 solution portions outgoing from the chamber is reported in the following table:

| Portions of STPP 0.5% | $Ca^{++}$ mg |
|---|---|
| 1 | 9.9 |
| 2 | 9.8 |
| 3 | 9.8 |
| 4 | 9.2 |
| 5 | 9.0 |

Therefore, in total the water solution in continuous lavage modality removed:

$$9.9+9.8+9.8+9.2+9.0 = 47.7\ Ca^{++}\ mg$$

corresponding to:

$$47.7/157.4 = \mathbf{30.3\%\ of\ calcium\ brought\ in\ solution}$$

*Dissolving solution: Sodium tripolyphosphate 0.5% - Salt to be dissolved: hydroxyapatite Modality: Step-wise lavage*

The outgoing calcium found in the 5 solution portions outgoing from the chamber is reported in the following table:

| Portions of STPP 0.5% | $Ca^{++}$ mg |
|---|---|
| 1 | 3.7 |
| 2 | 3.5 |
| 3 | 3.4 |
| 4 | 3.2 |
| 5 | 3.2 |

Therefore, in total the water solution in continuous lavage modality removed:

$$3.7+3.5+3.4+3.2+3.2 = 17.0\ Ca^{++}\ mg$$

corresponding to:

$$17.0/199.1 = \mathbf{8.5\%\ of\ calcium\ brought\ in\ solution}$$

The results are summarized in the following table:

| Dissolving solution | Salt to be dissolved | Modality | % calcium brought in solution |
|---|---|---|---|
| Water | Calcium pyrophosphate | Continuous lavage | 0.27 |

(continued)

| Dissolving solution | Salt to be dissolved | Modality | % calcium brought in solution |
|---|---|---|---|
| Water | Hyd roxyapatite | Continuous lavage | 0.10 |
| SMP 0.5% | Calcium pyrophosphate | Continuous lavage | 34.4 |
| SMP 0.5% | Hydroxyapatite | Continuous lavage | 9.9 |
| STPP 0.5 % | Calcium pyrophosphate | Continuous lavage | 27.6 |
| STPP 0.5 % | Hydroxyapatite | Continuous lavage | 7.1 |
| Water | Calcium pyrophosphate | Step-wise lavage | 0.30 |
| Water | Hydroxyapatite | Step-wise lavage | 0.15 |
| SMP 0.5% | Calcium pyrophosphate | Step-wise lavage | 52.5 |
| SMP 0.5% | Hydroxyapatite | Step-wise lavage | 11.4 |
| STPP 0.5 % | Calcium pyrophosphate | Step-wise lavage | 37.2 |
| STPP 0.5 % | Hydroxyapatite | Step-wise lavage | 8.5 |

Comparison with previous data

The same comparison has been carried out using the prior art described in the previous patent application No, WO2005/060978**,** to check whether a sodium metaphosphate solution or a sodium triphosphate solution could dissolve salts of calcium pyrophosphate and hydroxyapatite in "static" fashion.

50 mg of the two of calcium salts to be dissolved with a 50 ml solution of SMP 0.5% or with a 50 ml solution di STPP 0.5% were placed in a beaker. The mixture was put under stirring for 60 minutes and held at 37°C by thermostatic bath.

The results obtained by operating according to the mentioned patent application are reported in the following table:

| Dissolving solution | Salt to be dissolved | Modality | % calcium brought in solution |
|---|---|---|---|
| SMP 0.5% | Calcium pyrophosphate | Static solubilization | 30.2 |
| SMP 0.5% | Hydroxyapatite | Static solubilization | 8.5 |
| STPP 0.5 % | Calcium pyrophosphate | Static solubilization | 25.3 |
| STPP 0.5 % | Hydroxyapatite | Static solubilization | 6.9 |

As can be seen from the above two tables for calcium pyrophosphate the solubilization in static lavage modality by SMP is 30.2% compared to 34.4% in continuous lavage modality, to reach 52.5% in step-wise lavage modality.

For hydroxyapatite the solubilization by STP in static lavage modality is 8.5% compared to 9.9% in continuous lavage modality, to reach 11.4 in step-wise lavage modality.

As concerns STPP, for calcium pyrophosphate the solubilization in static lavage modality by STPP is 25.3% compared to 27.6% in continuous lavage modality, to reach 37.2% in step-wise lavage modality.

For hydroxyapatite the solubilization by STPP in static lavage modality is 6.9% compared to 7.1% in continuous lavage modality, to reach 8.5 in step-wise lavage modality.

The above shows that in continuous lavage and step-wise lavage modality the solubility of the two calcium salts is greater than in the static lavage modality, because a mechanical removal effect of the two salts is added to the effect of chemical solubilization from Safedia 0.5% and STPP 0,5% solutions.

## Claims

**1.** A preparation for use in the treatment of arthropathies due to microcrystal deposition, comprising a diluent aqueous sterile solution and a linear polymetaphosphate of the formula $M_{n+2}P_nO_{3n+1}$, or a cyclic polymetaphosphate of the formula $(MPO_3)_n$, wherein M represents an alkaline metal, as sterile lyophilized powder, wherein the volume of

diluent solution is from 100 ml to 1500 ml and the weight/volume ratio of the lyophilized polymetaphosphate powder to the diluent solution is from 1:1000 to 10:1000, and wherein said treatment is carried out, upon reconstitution of the polymetaphosphate powder in said diluent solution,

a) by step-wise lavage, i.e. by using, for an articular lavage, a plurality of defined portions of said reconstituted solution, in sequence; or
b) by a continuous lavage with said reconstituted solution,

wherein said polymetaphosphate is selected from polymeric metaphosphate, tripolymetaphosphate and cyclic hexametaphosphate.

2. A preparation for the use according to claim 1, wherein the polymetaphosphate is a sodium salt of the formula $(NaPO_3)_n$ or $Na_{n+2}P_nO_{3n+1}$.

3. A preparation for the use according to claim 2, wherein the polymetaphosphate is selected from the group consisting of: sodium polymeric metaphosphate (SMP), sodium tripolymetaphosphate (PSTP or STPP), sodium cyclic hexametaphosphate (SHMP).

4. A preparation for the use according to claim 3, wherein polymetaphosphate is sodium cyclic hexametaphosphate (SHMP).

5. A preparation for the use according anyone of claims 1 to 4, including further effective amounts of antioxidants and/or anti-free radical agents.

6. A preparation for the use according to claim 5, wherein the antioxidants are selected from the group consisting of: mannitol, vitamin E, vitamin C, carotenoids, tocopherol, taurine, glucosamine sulfate, glucosamine hydrochloride.

7. A preparation for the use according to any one of the previous claims, wherein the weight/volume ratio of the lyophilized polymetaphosphate powder to the diluent solution is from 3:1000 to 7:1000.

8. A preparation for the use according to claim 7, wherein said weight/volume ratio is 5:1000.

9. A preparation for the use according to any one of the previous claims, wherein the diluent solution volume is comprised from 300 ml and 1000 ml.

10. The preparation for the use according to claim 9, wherein said volume is equal to 500 ml.

11. The preparation for the use according to claim 4, wherein the weight/volume ratio of the lyophilized polymetaphosphate powder to the diluent solution is 5:1000 and the diluent solution volume is 500 ml.

12. The preparation for the use according to claim 11, wherein said diluent solution comprises mannitol and phosphate buffer.

## Patentansprüche

1. Präparat zur Anwendung in der Behandlung von Arthropathien aufgrund von Mikrokristallablagerung, umfassend eine wässrige sterile Verdünnungslösung und ein lineares Polymetaphosphat der Formel $M_{n+2}P_nO_{3n+1}$ oder ein cyclisches Polymetaphosphat der Formel $(MPO_3)_n$, wobei M ein Alkalimetall darstellt, als steriles lyophilisiertes Pulver, wobei das Volumen der Verdünnungslösung 100 ml bis 1500 ml beträgt und das Gewichts-/Volumen-Verhältnis des lyophilisierten Polymetaphosphatpulvers zu der Verdünnungslösung 1:1000 bis 10:1000 beträgt, und wobei die Behandlung bei Rekonstitution des Polymetaphosphatpulvers in der Verdünnungslösung durchgeführt wird,

a) durch schrittweise Spülung, d.h. durch Verwenden einer Vielzahl definierter Teile der rekonstituierten Lösung nacheinander für eine Gelenkspülung; oder
b) durch eine kontinuierliche Spülung mit der rekonstituierten Lösung,

wobei das Polymetaphosphat ausgewählt ist aus polymerem Metaphosphat, Tripolymetaphosphat und cyclischem Hexametaphosphat.

2. Präparat zur Anwendung nach Anspruch 1, wobei das Polymetaphosphat ein Natriumsalz der Formel $(NaPO_3)_n$ oder $Na_{n+2}P_nO_{3n+1}$ ist.

3. Präparat zur Anwendung nach Anspruch 2, wobei das Polymetaphosphat aus der folgenden Gruppe ausgewählt ist: Polymeres Natriummetaphosphat (SMP), Natriumtripolymetaphosphat (PSTP oder STPP), cyclisches Natriumhexametaphosphat (SHMP).

4. Präparat zur Anwendung nach Anspruch 3, wobei das Polymetaphosphat cyclisches Natriumhexametaphosphat (SHMP) ist.

5. Präparat zur Anwendung nach einem der Ansprüche 1 bis 4, weiterhin enthaltend wirksame Mengen an Antioxidantien und/oder Wirkstoffe gegen freie Radikale.

6. Präparat zur Anwendung nach Anspruch 5, wobei die Antioxidantien aus der Gruppe ausgewählt sind, die aus: Mannitol, Vitamin E, Vitamin C, Carotinoiden, Tocopherol, Taurin, Glucosaminsulfat und Glucosaminhydrochlorid besteht.

7. Präparat zur Anwendung nach einem der voranstehenden Ansprüche, wobei das Gewichts-/Volumen-Verhältnis des lyophilisierten Polymetaphosphatpulvers zu der Verdünnungslösung 3:1000 bis 7:1000 beträgt.

8. Präparat zur Anwendung nach Anspruch 7, wobei das Gewichts-/VolumenVerhältnis 5:1000 ist.

9. Präparat zur Anwendung nach einem der voranstehenden Ansprüche, wobei das Verdünnungslösungsvolumen 300 mL bis 1000 ml umfasst.

10. Präparat zur Anwendung nach Anspruch 9, wobei das Volumen 500 ml beträgt.

11. Präparat zur Anwendung nach Anspruch 4, wobei das Gewichts-/VolumenVerhältnis des lyophilisierten Polymetaphosphatpulvers zu der Verdünnungslösung 5:1000 beträgt und das Verdünnungslösungsvolumen 500 ml beträgt.

12. Präparat zur Anwendung nach Anspruch 11, wobei die Verdünnungslösung Mannitol und Phosphatpuffer umfasst.

## Revendications

1. Préparation pour son utilisation dans le traitement d'arthropathies dues à un dépôt de microcristaux, comprenant une solution aqueuse stérile à rôle de diluant et un polymétaphosphate linéaire de formule $M_{n+2}P_nO_{3n+1}$, ou un polymétaphosphate cyclique de formule $(MPO_3)_n$, dans laquelle M représente un métal alcalin, sous la forme d'une poudre lyophilisée stérile, dans laquelle le volume de solution à rôle de diluant est de 100 ml à 1500 ml et le rapport poids/volume de la poudre de polymétaphosphate lyophilisée sur la solution à rôle de diluant est de 1:1000 à 10:1000, et dans laquelle ledit traitement est réalisé, après la reconstitution de la poudre de polymétaphosphate dans ladite solution à rôle de diluant,

   a) par lavage par étapes, en d'autres termes en utilisant, pour un lavage articulaire, une pluralité de parties définies de ladite solution reconstituée, en séquence ; ou
   b) par un lavage continu avec ladite solution reconstituée,

   dans laquelle ledit polymétaphosphate est sélectionné parmi le métaphosphate polymère, le tripolymétaphosphate et l'hexamétaphosphate cyclique.

2. Préparation pour son utilisation selon la revendication 1, dans laquelle le polymétaphosphate est un sel de sodium de formule $(NaPO_3)_n$ ou $Na_{n+2}P_nO_{3n+1}$.

3. Préparation pour son utilisation selon la revendication 2, dans laquelle le polymétaphosphate est sélectionné dans le groupe consistant en: le métaphosphate polymère de sodium (SMP), le tripolymétaphosphate de sodium (PSTP

ou STPP), l'hexamétaphosphate cyclique de sodium (SHMP).

**4.** Préparation pour son utilisation selon la revendication 3, dans laquelle le polymétaphosphate est l'hexamétaphosphate cyclique de sodium (SHMP).

**5.** Préparation pour son utilisation selon l'une quelconque des revendications 1 à 4, comprenant en outre des quantités efficaces d'antioxydants et/ou d'agents anti-radicaux libres.

**6.** Préparation pour son utilisation selon la revendication 5, dans laquelle les antioxydants sont sélectionnés dans le groupe consistant en : le mannitol, la vitamine E, la vitamine C, les caroténoïdes, le tocophérol, la taurine, le sulfate de glucosamine, le chlorhydrate de glucosamine.

**7.** Préparation pour son utilisation selon l'une quelconque des revendications précédentes, dans laquelle le rapport poids/volume de la poudre de polymétaphosphate lyophilisée sur la solution à rôle de diluant est de 3:1000 à 7:1000.

**8.** Préparation pour son utilisation selon la revendication 7, dans laquelle ledit rapport poids/volume est de 5:1000.

**9.** Préparation pour son utilisation selon l'une quelconque des revendications précédentes, dans laquelle le volume de la solution à rôle de diluant est compris entre 300 ml et 1000 ml.

**10.** Préparation pour son utilisation selon la revendication 9, dans laquelle ledit volume est égal à 500 ml.

**11.** Préparation pour son utilisation selon la revendication 4, dans laquelle le rapport poids/volume de la poudre de polymétaphosphate lyophilisée sur la solution à rôle de diluant est de 5:1000 et le volume de la solution à rôle de diluant est de 500 ml.

**12.** Préparation pour son utilisation selon la revendication 11, dans laquelle ladite solution à rôle de diluant comprend du mannitol et un tampon phosphate.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2005060978 A

### Non-patent literature cited in the description


- **MCCARTHY DJ JR et al.** The significance of calcium phosphate crystal in the synovial fluid of arthritis patients: the pseudogout syndrome. Clinical aspects. *Ann Intern Med,* 1962, vol. 56, 711-737
- **DIEPPE PA et al.** Apatite deposition disease: a new arthropathy. *Lancet,* 1976, vol. 1, 266-268
- Calcium Pyrophosphate Crystal Deposition Disease; Pseudogout; Articular Chondrocalcinosis. **RYAN ML et al.** Arthritis and Allied Conditions: A Textbook of Rheumatology. Lippincott Williams & Wilkins, 1993, 1835-1855
- **ANDERSON HC.** Mechanisms of pathologic calcification. *Rheum Dis Clin N Am,* 1988, vol. 14, 303-319
- **BURT HM ; JACKSON JK.** Enhancement of crystal induced neutrophil responses by opsonisation of calcium pyrophosphate dihydrate crystals. *Ann Rheum Dis,* 1993, vol. 52, 599-607
- **SHWAN A et al.** Comparison of sodium urate and calcium pyrophosphate crystal size and other factors. *Arthritis Rheum,* 1995, vol. 18, 783-793
- **FITZGERALD RH JR.** Intrasynovial injection of steroids uses and abuses. *Mayo Clin Proc,* 1976, vol. 51, 655-659
- **WERLEN O et al.** Corticosteroid therapy for the treatment of acute attacks of crystal-induced arthritis: an effective alternative to nonsteroidal antiinflammatory drugs. *Rev Rhum Engl Ed,* 1996, vol. 63, 248-254
- **ABRAMSON SB.** Treatment of gout and crystal arthropathies and use and mechanisms of action of nonsteroidal anti-inflammatory drugs. *Curr Opin Rheumatol,* 1992, vol. 4, 295-300
- **GONZALES T ; GANTES M.** Prevention of acute attacks of pseudogout with oral colchicine. *J Rheumatol,* 1987, vol. 14, 632-633
- **LANGE U et al.** Current aspects of colchicine therapy - classical indications and new therapeutic uses. *Eur J Med Res,* 2001, vol. 6, 150-160
- **XU Y et al.** Effects of pyrophosphatase on dissolution of calcium pyrophosphate dihydrate crystals. *J Rheumatol,* 1991, vol. 18, 66-71
- **SHINOZAKI T et al.** Calcium pyrophosphate dihydrate (CPPD) crystal dissolution by alkaline phosphatase: interaction of alkaline phosphatase on CPPD crystals. *J Rheumatol,* 1995, vol. 22, 117-123
- **COSENTINO R. et al.** Extracorporeal shock wave therapy for chronic calcific tendinitis of the shoulder: single blind study. *Ann Rheum Dis,* 2003, vol. 62, 248-50
- **EBENBICHLER GR et al.** Ultrasound therapy for calcific tendinitis of the shoulder. *N Engl J Med,* 1999, vol. 341, 1237
- **CINI R. et al.** Dissolution of calcium pyrophosphate crystals by polyphosphates: an in vitro and ex vivo study. *Ann Rheum Dis,* 2001, vol. 60, 962-967